# EUROPEAN PATENT APPLICATION

(11) **EP 1 935 484 A2**
(43) Date of publication of application: **25.06.2008**
(21) Application number: 07023941.3
(22) Date of filing: 11.12.2007
(51) Int. Cl.: B01J 19/00, C12Q 1/68, G01N 33/551

(54) **Oligomer probe array chip, related mask and analysis method**

(30) Priority: 20.12.2006 KR 20060131208
(71) Applicant: Samsung Electronics Co., Ltd., Suwon-si, Gyeonggi-Do (KR)
(72) Inventor: Hah, Jung-Hwan, Osaka Prefacture, 560-0083 (JP); Chi, Sung-Min, Hwaseong-shi, Gyeonggi-do (KR); Kim, Kyoung-Seon, Yeongtong-gu,Suwon-shi, Gyeonggi-do (KR); Kim, Won-Sun, Yeongtong-gu, Suwon-shi, Gyeonggi-do (KR); Cho, Han-Ku, Seongnam-si, Gyeonggi-do (KR)
(74) Representative: Patentanwälte Ruff, Wilhelm, Beier, Dauster & Partner

(57) **Abstract**

An oligomer probe array chip according to the invention includes a substrate and a main array on the substrate having a plurality of sub-arrays in rows or panels. The sub-arrays include a plurality of spots arranged in a matrix to which oligomer probes having different sequences may be attached. The invention further provides a mask for fabricating such oligomer probe array chip, and a hybridization analysis method of such oligomer probe array chip.

## Description

The invention relates to an oligomer probe array chip, to a mask for forming an oligomer probe array chip, and to a hybridization analysis method of an oligomer probe array chip.

An oligomer probe array chip may be used to perform genotyping, analyze proteins and/or peptides, screen drugs, and develop and produce new drugs using gene expression profiling and/or mutation and polymorphism detection including, for example, SNP.

A scan-by-pixel type of Photo-Multiplier Tube (PMT) scanner and a scan-by-area type of Charge-Coupled Device (CCD) scanner may be used to analyze the results of hybridization of an oligomer probe array chip.

In a PMT scanner, excitation and emission may be performed in pixel units as optic systems or chips move, and the PMT may amplify an emission value obtained based on the type of pixel to form final images through an A/D converter. In a PMT scanner, a PMT gain may be controlled to amplify light having a lower intensity, and sensitivity may be higher. PMT scanners may be used to detect fluorescence of an oligomer probe array chip having lower intensity. Because PMT scanners are a scan-by-pixel type, a laser having higher intensity corresponding to the excitation wavelength may be used to minimize dwell time for each pixel so that scan time may be reduced. If the type of fluorescent dye must be changed to perform various types of tests using the oligomer probe array chip, an additional laser may be required and the PMT and the optic system may require reconfiguration. It may be difficult to perform reconfiguration using a simple process, and the cost may be higher. Because PMT scanners are a scan-by-pixel type, one or more of the optic systems (for example, the lens and/or the laser) and the chips may need to move with precision in terms of the pixel unit. Because of these potential requirements, PMT scanners may have less component flexibility. Design complexity may be higher if high density oligomer probe array chips are analyzed.

CCD scanners' pixel resolution may depend on the area of the CCD chip, the pixel size, and/or magnification of the lighting system, and it may be easier to improve pixel resolution compared to PMT scanners.

Because CCD scanners are scan-by-area type, the scanning dimension may be controlled in an area unit having hundreds of thousands or millions pixels, compared to a single pixel unit used by scan-by-pixel type scanners. Thus, scanning pitch may be increased, causing discontinuity between adjacent scanning images. Discontinuity may increase with increased pixel resolution.

It is the technical problem underlying the invention to provide an oligomer probe array chip, a corresponding mask for forming such chip, and a hybridization analysis method of such chip which are capable to reduce or avoid the inconveniencies of the prior art mentioned above.

The invention solves this problem by providing an oligomer probe array chip having the features of claim 1, a mask having the features of claim 9, and a hybridization analysis method having the features of claim 16. Advantageous embodiments of the invention are mentioned in the dependent claims, the wording of which is herewith incorporated by reference to avoid unnecessary text repetition.

Example embodiments may provide an oligomer probe array chip including a substrate, a main array on the substrate having a plurality of sub-arrays in rows, and/or a plurality of sub-array alignment spot arrays outside of each of the sub-array rows. The sub-arrays each may include a plurality of spots in a matrix to which oligomer probes of different sequences may be fixed.

Example embodiments may provide an oligomer probe array chip including a substrate and a main array on the substrate having a plurality of sub-arrays in panels separated from each other by cross-shaped spaces in a matrix. The panel type sub-arrays may each include a plurality of spots in a matrix to which oligomer probes having different sequences may be fixed.

Example embodiments may provide a mask having a main array pattern for forming oligomer probe array chips, the mask including a plurality of sub-array patterns in rows and/or a plurality of sub-array pattern alignment spot array patterns outside of each sub-array row pattern. The sub-array patterns each may include a plurality of spot patterns in a matrix.

Example embodiments may provide a mask having a main array pattern for forming an oligomer probe array chip, the mask including a plurality of panel type sub-array patterns separated from each other by cross-shaped spaces in a matrix.

Example methods may provide a hybridization analysis method of an oligomer probe array chip. Example methods may include hybridizing a target sample and/or an oligomer probe array chip with a plurality of sub-arrays in rows, forming images of all sub-arrays on the probe by repeatedly determining a position of the sub-array by the sub-array alignment spot array, forming an image of each sub-array by, for example, a TDI type CCD scanner, and aligning the obtained sub-array images to form a single hybridization image.

Example methods may provide a hybridization analysis method including hybridizing a target sample and/or an oligomer probe array chip with a plurality of sub-arrays in panels separated from each other by cross-shaped spaces in a matrix, forming images of all the sub-arrays by repeatedly determining a position of each of the sub-arrays by the spaces, forming an image of each of the sub-arrays by, for example, a CCD scanner, and aligning the obtained sub-array images to form a single hybridization image.

Advantageous embodiments of the invention are described in the following and shown in the drawings in which:

FIG. 1 is a layout of an example embodiment mask that may be used to produce an oligomer probe array chip;

FIG. 2 is a layout that illustrates a relationship between a CCD width of a Time Delay Integration (TDI) type of scanner and a sub-array in rows;

FIG. 3 is a sectional view of the example embodiment oligomer probe array chip that may be produced using the mask shown in FIG. 1;

FIG. 4 is a flowchart illustrating an example hybridization analysis of an oligomer probe array chip;

FIG 5 is a layout of an alternative example embodiment mask that may be used to produce an oligomer probe array chip;

FIG. 6 is a layout of an alternative example embodiment mask that may be used to produce an oligomer probe array chip;

FIG. 7 is a layout that illustrates a relationship between a CCD width of a step-and-repeat type of scanner and a panel type of sub-array;

FIG. 8 is a sectional view of an example embodiment oligomer probe array chip that may be produced using the mask shown in FIG. 6; and

FIG. 9 is a flowchart illustrating an example hybridization analysis of an oligomer probe array chip.

Various example embodiments of the invention will now be described more fully with reference to the accompanying drawings. In the drawings, the thicknesses of layers and regions may be exaggerated for clarity. Detailed illustrative embodiments of the present invention are disclosed herein. However, specific structural and functional details disclosed herein are merely representative for purposes of describing example embodiments of the present invention. This invention may, however, may be embodied in many alternate forms and should not be construed as limited to only the embodiments set forth herein. Like numbers refer to like elements throughout the description of the figures.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Other words used to describe the relationship between elements should be interpreted in a like fashion (e.g., "between" versus "directly between", "adjacent" versus "directly adjacent", etc.).

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

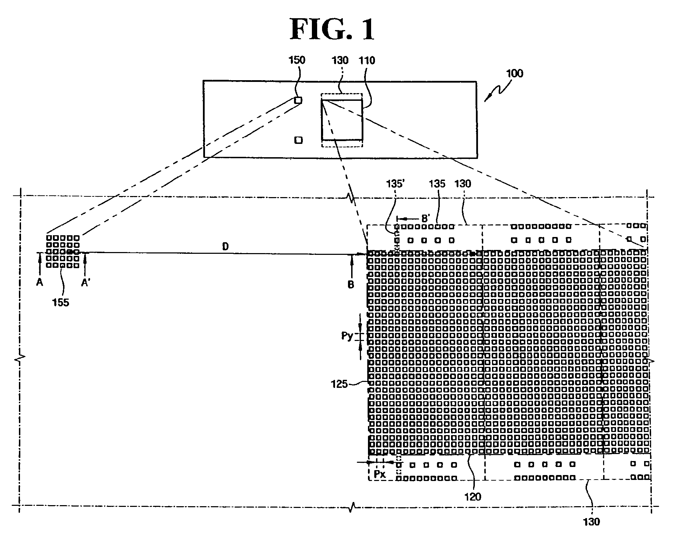

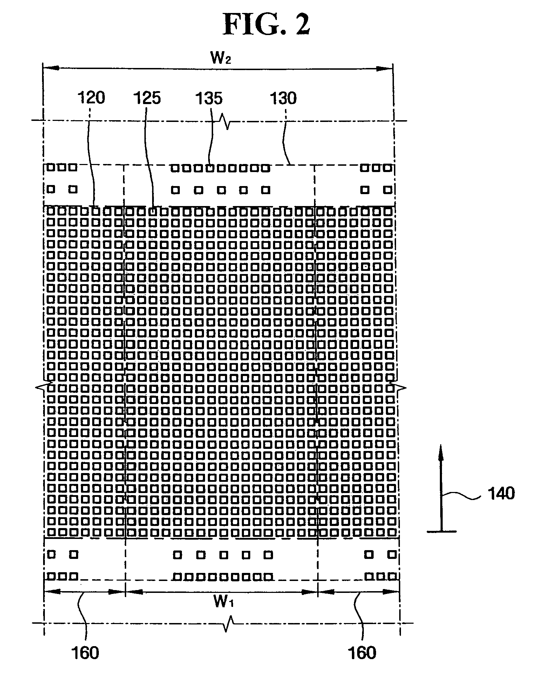

FIG. 1 is a layout of an example embodiment mask that may be used to produce an oligomer probe array chip. FIG. 2 is a layout that illustrates a relationship between a CCD width of a scanner and a sub-array row. FIGS. 1 and 2 show the layouts of a mask that may be used to produce an oligomer probe array chip compatible with a TDI type of CCD scanner.

As shown in FIGS. 1 and 2, an example mask 100 for producing example embodiment oligomer probe array chips may include a main array pattern 110. The main array pattern 110 may include of a plurality of sub-array patterns 120 in rows. Sub-array pattern alignment spot array patterns 130 may be on outer sides of each of the sub-array patterns 120. A plurality of spot patterns 125 may be arranged in an M x N matrix, with N ≥ M, in the sub-array pattern 120. A plurality of spot patterns 125 may be arranged at pitches Px and Py. The x-direction pitch Px and the y-direction pitch Py may be substantially the same as or different from each other. The sub-array pattern alignment spot array pattern 130 may be useful for automatic segmentation alignment if the sub-array patterns 120 form sides of the sub-array pattern alignment spot array pattern in a longitudinal direction (Y direction). The longitudinal direction may be a scanning transfer direction 140 of the example oligomer probe array chip. A plurality of spot patterns 135 may form a sub-array pattern alignment spot array pattern 130 by having a pitch that is the integer multiple of the y-direction pitch Py (e.g., m x Py, m ≥ 2) of the spot pattern 125 forming the sub-array pattern 120. FIG. 1 illustrates an arrangement of the spot patterns 135 in which virtual spot patterns 135' may be between the spot patterns 135 so that the pitch may be twice as large as Py.

As shown in FIG. 2, width W1 of the sub-array pattern 120 may be smaller than the width W2 of the CCD to align sub-array images. The width W2 of the CCD may be the product of a unit pixel pitch of the CCD scanner and the number of CCD pixels in the width W1 direction that is perpendicular to the scanning transfer direction 140 of the CCD scanner.

Because the width W1 of the sub-array pattern 120 may be smaller than the width W2 of the CCD, adjacent sub-array patterns 120 may overlap the CCD in an area 160 in the rotation tolerance range of the CCD scanner and permit double scanning and image alignment.

Global alignment spot array patterns 150 may be outside of the main array pattern 110 to globally align the main array pattern 110. Each of the global alignment spot array patterns 150 may be spaced from the main array pattern 110 by a distance D that is larger than the width W1 of the sub-array pattern 120 to improve precision of the global alignment. Each of the global alignment spot array patterns 150 may be formed of spot patterns 155 with the same pitch as the spot patterns 125 forming the sub-array pattern 120.

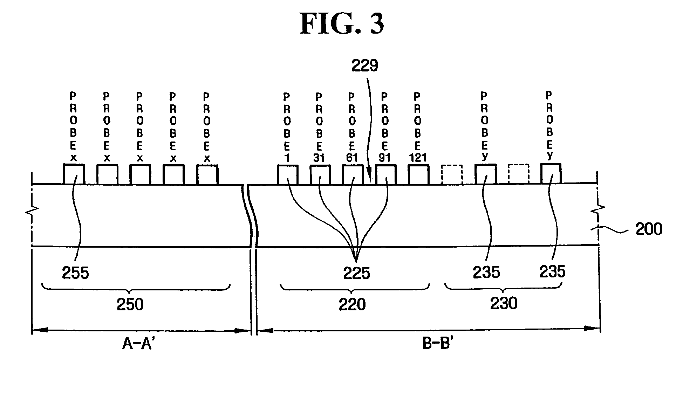

FIG 3 is a sectional view of an example embodiment oligomer probe array chip that may be fabricated with the example embodiment mask shown in FIG. 1.

As shown in FIG 3, a main array may include a plurality of sub-arrays 220 in rows on a substrate 200. Oligomer probes (PROBE1. PROBE31, PROBE61, etc.) having different sequences may be attached to a plurality of spots 225 in the sub-arrays 220. An oligomer probe having a sequence that is substantially different from the oligomer probes on spots 225 may be attached to the spot 235 (PROBEy) in the sub-array alignment spot array 230. Oligomer probes attached to the spots 235 may have the same sequence. An oligomer probe having a sequence different from the oligomer probe of the spot 225 may be attached to the spot 255 (PROBEx) in the global alignment spot array 250. Oligomer probes attached to the spots 255 may have the same sequence.

As discussed in co-owned Korean Patent Application Nos. 2006-0039713 and 2006-0039716, which are incorporated herein in their entirety by reference, spots 225, 235, and/or 255 of FIG. 3 may be probe cell actives separated by a probe cell separation region 229 that does not contain a functional group coupled with an oligomer probe.

Spots 225, 235, and/or 255 may be active regions on the substrate instead of the probe cell active, and regions other than spots 225, 235, and 255 may be deactivated regions on the substrate.

Oligomers may include a polymer having two or more monomers covalently bonded to each other. Monomers may have a molecular weight of about 1000 or less, and the polymer may have any molecular weight. The oligomer may contain about 2 to about 500 monomers, for example, about 5 to about 30 monomers. Example monomers may include nucleosides, nucleotides, amino acids, and/or peptides according to the type of probe. Oligomer probes synthesized in advance may be used, or oligomer probes may be synthesized on the spots 225, 235, and 255 using an in-situ photolithography process or other suitable process.

Nucleosides and/or nucleotides may contain purine and pyrimidine bases, methylated purine or pyrimidine, and/or acylated purine or pyrimidine. Nucleosides and/or nucleotides may contain ribose and deoxyribose sugars, or denaturalized sugars in which one or more hydroxyl groups may be substituted with halogen atoms or aliphatics containing functional groups such as ethers or amines.

In the substrate 200, non-specific binding may be minimized or prevented during the hybridization process, and the substrate 200 may be transparent with respect to visible rays and/or UV. The substrate 200 may be a flexible or rigid substrate. For example, flexible substrates may include membrane and/or plastic films made of nylon and/or nitrocellulose, and rigid substrates may include a silicon substrate and/or a transparent glass substrate such as soda lime glass. If a silicon substrate or the transparent glass substrate is used, non-specific binding may not occur or occur less during the hybridization process. If a transparent glass substrate is used, it may be more feasible to detect fluorescent substances because a glass substrate may be transparent in respect to the visible rays and/or UV rays. The silicon substrate and/or the transparent glass substrate may be compatible with processes of producing thin films and/or known photolithography processes for producing semiconductor devices or LCD panels without being modified.

Spots 225, 235, and 255 may not be hydrolyzed but may be substantially stable during hybridization analysis, for example, if spots come into contact with a phosphoric acid having a pH of about 6 to about 9 and/or a TRIS buffer. Spots may be made of a silicon oxide film, for example, a PE-TEOS film, an HDP oxide film, a P-SiH₄ oxide film, a thermal oxide film, and/or another suitable silicon oxide film. Spots may be made from silicates, for example, hafnium silicate, zirconium silicate, and/or another suitable silicate. Spots may be made from a metal oxynitride film, for example, a silicon oxynitride film, a hafnium oxynitride film, a zirconium oxynitride film, and/or another suitable oxynitride film. Spots may be made from a metal oxide film, for example, titanium oxide film, a tantalum oxide film, an aluminum oxide film, a hafnium oxide film, a zirconium oxide film, an ITO, and/or another suitable metal oxide film polyimide. Spots may be made from polyamine. Spots may be made from metals, for example, gold, silver, copper, palladium, and/or another suitable metal. Spots may be made from a polymer, for example, polystyrene, a polyacrylic acid, polyvinyl, and/or another suitable polymer. Spots may be made from any combination of the above listed materials.

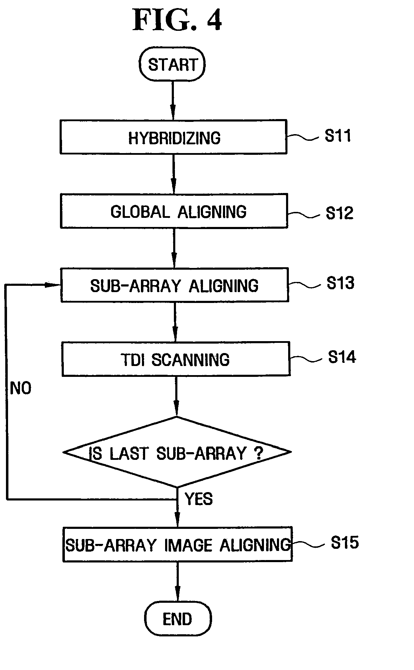

FIG. 4 is a flowchart illustrating an example method of hybridization analysis on an oligomer probe array chip. The example method is described with reference to the example embodiment oligomer probe array chip 200 in FIG. 3.

Hybridization of a target sample may be performed on the oligomer probe array chip 200 (S11). After hybridization, the oligomer probe array chip 200 may be mounted on the TDI type of CCD scanner, and global alignment may be performed (S12). Position of the global alignment spot array 250 may be measured using the global alignment, and the measured value may be compared to a known reference value to determine the position of the main array 210. Position of the sub-array alignment spot array 230 may be measured, and the measured value and a known reference value may be compared to each other to determine the position of the first sub-array 220 (S 13). The TDI type of scanning involving chip transfer in the Y-direction and charge transfer in the negative Y-direction may be performed to form a sub-array image (S 14). Determining of the sub-array (S13) position and formation of the scanning image (S 14) may be repeated until the last sub-array 220 is formed. Because width W1 of the sub-array 220 may be smaller than the width W2 of the CCD, one or more spot columns may overlap adjacent sub-arrays 220 during scanning (S14). Alignment and analysis of the sub-array images (S 15) may be performed once the last sub-array 220 is formed. Sub-array images obtained using doubly scanned spots may be aligned to form a single image, and results of the hybridization between the probe and the target sample may be obtained according to the scanned spot.

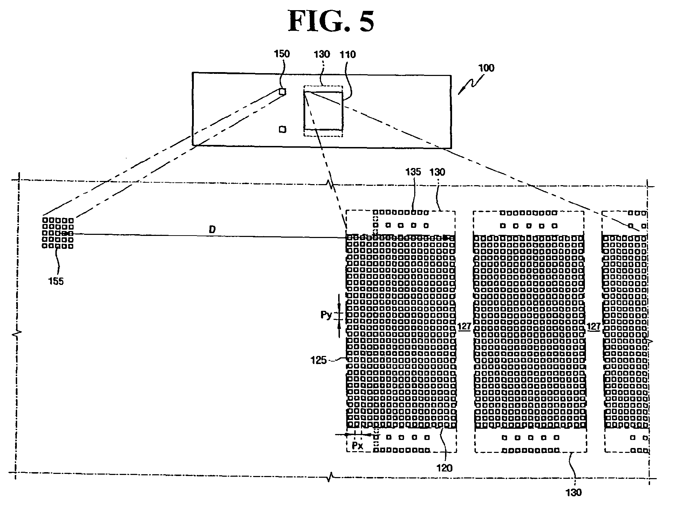

FIG. 5 is a layout of a mask that may be used to produce an example embodiment oligomer probe array chip.

The example embodiment mask of FIG. 5 may be different from the example embodiment mask of FIG. 1 in that a space 127 may be between the sub-arrays 120, but other constituent elements may be substantially the same between the masks of FIGS. 1 and 5 and a description of redundant elements is omitted.

The mask shown in FIG. 5 may be used to produce an oligomer probe array chip having integration clearance, space 127 and the sub-array pattern alignment spot array 130 may be used as alignment keys.

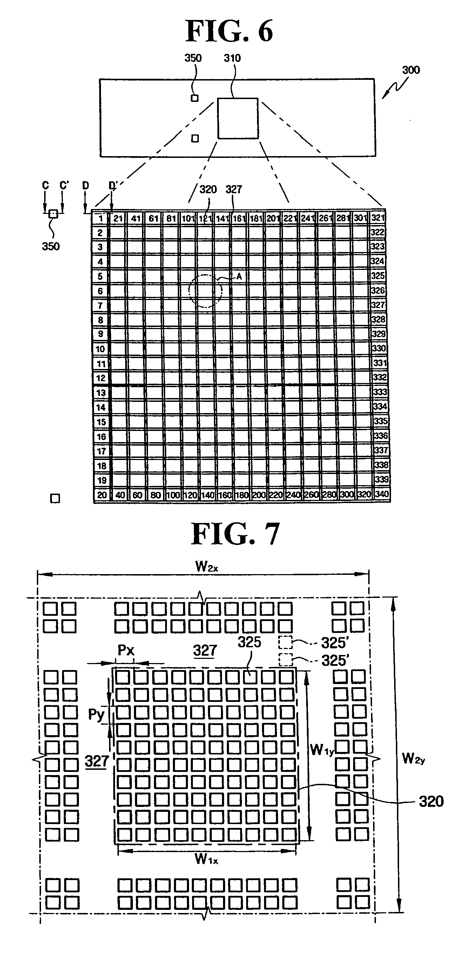

FIG. 6 is a layout of an example embodiment mask that may be used to produce an example embodiment oligomer probe array chip. FIG. 7 is an enlarged view of a portion A of FIG. 6 and illustrates the relationship between a CCD width and a panel type of sub-array. FIGS. 6 and 7 are layouts of example embodiment masks used to produce an oligomer probe array chip compatible with a step and repeat type of CCD scanner.

As shown in FIGS. 6 and 7, a mask 300 for producing example embodiment oligomer probe array chips may include a main array pattern 310. The main array pattern 310 may be formed of a plurality of panel type sub-array patterns 320 that are separated from each other by cross-shaped spaces 327. A plurality of spot patterns 325 may be in a matrix in the sub-array pattern 320. A plurality of spot patterns 325 may be spaced with pitches Px and Py. The x-direction pitch, Px, and the y-direction pitch, Py, may be substantially similar, and the number of x-direction spot patterns 325 and the number of y-direction spot patterns 325 may be the substantially the same. The space 327 may be used as an alignment standard if the space 327 is larger than the pitch of the spot pattern 325. FIGS. 6 and 7 illustrate the arrangement of two virtual spot patterns 325'.

As shown in FIG. 7, to perform image alignment, the x-axis direction width W1x and the y-axis direction width W1y of the panel type of sub-array pattern 320 may be smaller than the x-axis direction width W2x and the y-axis direction width W2y of the CCD. The width W2x of the CCD in an x-axis direction may be the product of a unit pixel pitch of the CCD scanner in the x-axis direction and the number of pixels in the x-axis direction. The width W2y of the CCD in an y-axis direction may be the product of a unit pixel pitch of the CCD scanner in the y-axis direction and the number of pixels in the y-axis direction. As described above, because widths W1x and W1y of the sub-array pattern 320 may be smaller than the widths W2x and W2y of the CCD, respectively, the adjacent sub-array patterns 320 may be scanned twice in the rotation tolerance range of a CCD scanner to facilitate image alignment.

Global alignment spot array patterns 350 may be outside of the main array pattern 310 to perform global alignment of the main array pattern 310. Each of the global alignment spot array patterns 350 may be spaced from the main array pattern 310 by a distance larger than width W1x of the sub-array pattern 320 to improve precision of global alignment. Each of the global alignment spot array patterns 350 may be formed of spot patterns (not shown) arranged with similar pitches as the spot pattern 325 forming the sub-array pattern 320.

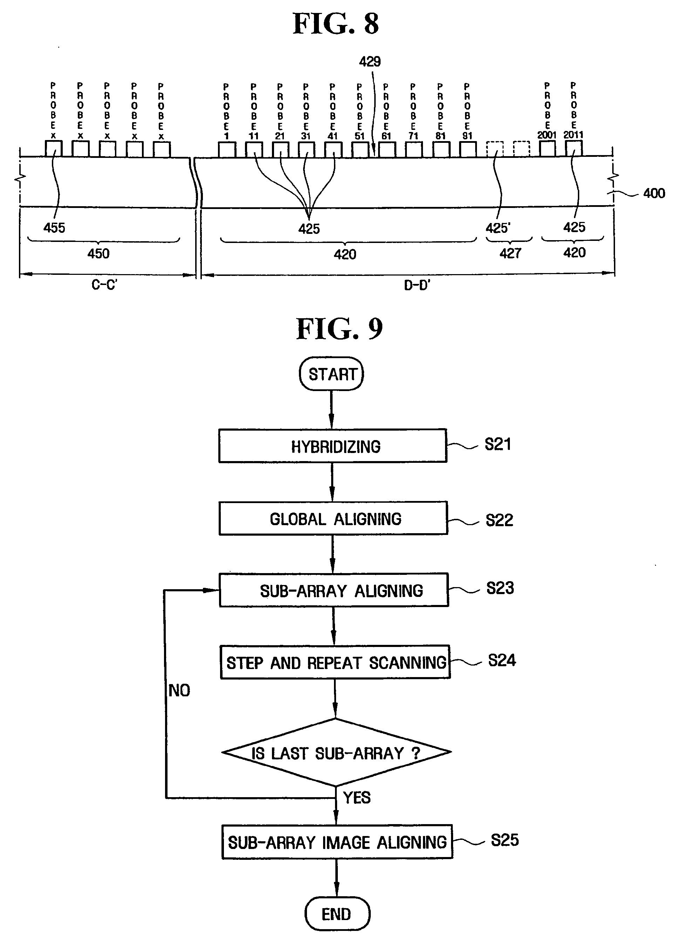

FIG. 8 is a sectional view of an example embodiment oligomer probe array chip that may be produced using an example embodiment mask shown in FIG. 6.

As shown in FIG. 8, a main array with a plurality of panel type sub-arrays 420 may be on a substrate 400. Oligomer probes (PROBE1, PROBE11, PROBE21, etc.) having different sequences may be attached to a plurality of spots 425 making up the main array. Oligomer probes (PROBEx) having similar sequences different from an oligomer probe attached to spots 425 may be attached to spot 455 making up the global alignment spot array 450. Oligomer probes that are fixed to the spots 455 may have the same sequence.

As discussed in co-owned Korean Patent Application Nos. 2006-0039713 and 2006-0039716, which are incorporated herein in their entirety by reference, spots 425 and/or 455 of FIG. 8 may be probe cell actives separated by a probe cell separation region 429 that does not contain a functional group coupled with an oligomer probe.

Spots 425 and/or 455 may be active regions on the substrate instead of the probe cell active, and regions other than spots 425 and 455 may be deactivated regions on the substrate.

FIG 9 is a flowchart illustrating a hybridization analysis method of the oligomer probe array chip. The example method is described with reference to the example embodiment oligomer probe array chip 400 in FIG. 8.

Hybridization of the target sample may be performed on the oligomer probe array chip 400 (S21). Oligomer probe array chip 400 may be mounted on a step-and-repeat type of CCD scanner, and global alignment may be performed (S22). Position of the global alignment spot array 450 may be measured using the global alignment, and the measured value may be compared to a known reference value to determine the position of the main array. Position of the cross-shaped space 427 may be measured, and the measured value and the reference value may be compared to determine the position of the first sub-array 420 (S23). The sub-array 420 may be focused and exposure time of the CCD may be modified to form a single panel type sub-array image (S4). Determining the position of the sub-array (S3) and formation of the scanning image (S4) may be repeated using the step and repeat process until the last sub-array 420 is formed. Because widths W1x and W1y of the sub-array pattern 320 may be smaller than the widths W2x and W2y of the CCD, one or more spot columns and spot rows may overlap adjacent sub-arrays 220 during the scanning. Alignment and analysis of the sub-array images (S25) may be performed after the final sub-array is formed. Sub-array images obtained using spots scanned twice may be aligned to form a single image, and the results of the hybridization of the probe and the target sample may be obtained according to the spot.

Using a CCD scanner in which resolution of a pixel may be better than in a PMT scanner due to transfer of the scanning stage and the scanning dimension being set in consideration of the layout of an oligomer probe array chip, it may be easier to analyze highly integrated data.

Although example embodiments have been described in connection with the attached drawings, it will be apparent to those skilled in the art that various modifications and changes may be made thereto without departing from the scope and spirit of the disclosure. Therefore, it should be understood that the above embodiments are not limitative, but illustrative in all aspects.

## Claims

1. An oligomer probe array chip comprising:
a substrate (200);
a main array (110) on the substrate, the main array including a plurality of sub-arrays (120) aligned in rows or panels and each of the sub-arrays including a plurality of spots (125) in a matrix form; and
a plurality of oligomer probes (PROBEx), each of the oligomer probes having a unique sequence and attached to a corresponding spot of the plurality of spots.

2. The oligomer probe array chip of claim 1, further comprising a plurality of sub-array alignment spot arrays (130) on the substrate outside each of the sub-arrays.

3. The oligomer probe array chip of claim 2, wherein a width of each of the sub-arrays is smaller than the product of a unit pixel pitch of a Charge-Coupled Device (CCD) scanner scanning the oligomer probe array chip and a number of CCD pixels in the width direction.

4. The oligomer probe array chip of claim 3, wherein the CCD scanner is a Time Delay Integration (TDI) type scanner or a step-and-repeat type scanner.

5. The oligomer probe array chip of any of claims 2 to 4, further comprising a plurality of global alignment spot arrays (150) separated from the plurality of sub-array alignment spot arrays and/or arranged outside of the main array.

6. The oligomer probe array chip of any of claims 1 to 5, wherein the sub-arrays are separated from each other by spaces.

7. The oligomer probe array chip of claim 6, wherein the sub-arrays are separated from each other by cross-shaped spaces to form a plurality of panels of sub-arrays.

8. The oligomer probe array chip of claim 7, wherein a first width of each of the panels of sub-arrays in an x-axis direction is smaller than a product of a unit pixel pitch of a Charge-Coupled Device (CCD) scanning the oligomer probe array chip in the x-axis direction and the number of CCD pixels in the x-axis direction and a second width of each of the panels of sub-arrays in a y-axis direction is smaller than a product of a unit pixel pitch of the CCD in the y-axis direction and the number of CCD pixels in the y-axis direction.

9. A mask for forming an oligomer probe array chip, the mask comprising:
a main array pattern (310) including a plurality of sub-array patterns (320) aligned in rows, each of the sub-array patterns including a plurality of spot patterns (325) in a matrix form; and
a plurality of sub-array pattern alignment spot array patterns outside of each of the sub-array patterns.

10. The mask of claim 9, wherein a width of each of the sub-array patterns is smaller than the product of a unit pixel pitch of a Charge-Coupled Device (CCD) scanner scanning the oligomer probe array chip and a number of CCD pixels in the width direction.

11. The mask of claim 10, wherein the CCD scanner is a Time Delay Integration (TDI) type scanner or a step-and-repeat type scanner.

12. The mask of any of claims 9 to 11, further comprising global alignment spot array patterns separated from the alignment spot array patterns and/or arranged outside of the main array pattern.

13. The mask of any of claims 9 to 12, wherein each of the sub array patterns are separated from each other by spaces (327).

14. The mask of claim 13, wherein each of the sub-array patterns are separated from each other by cross-shaped spaces so as to form a plurality of panels of sub-array patterns.

15. The mask of claim 14, wherein a first width of each of the panels of sub-array patterns in an x-axis direction is smaller than a product of a unit pixel pitch of a Charge-Coupled Device (CCD) scanner scanning the oligomer probe array chip in the x-axis direction and the number of CCD pixels in the x-axis direction and a second width of each of the panels of sub-arrays in a y-axis direction is smaller than a product of a unit pixel pitch of the CCD scanner in the y-axis direction and the number of CCD pixels in the y-axis direction.

16. A hybridization analysis method of an oligomer probe array chip, the hybridization analysis method comprising:
repeatedly determining a position of each sub-array of a plurality of sub-arrays of the oligomer probe array chip;
forming an image of each of the sub-arrays with a Charge-Coupled Device (CCD) scanner; and
aligning a plurality of the sub-array images to form a single hybridization image.

17. The method of claim 16, wherein repeatedly determining the position of each sub-array is based on a plurality of sub-array alignment spot arrays outside of a main array of the oligomer probe array chip and wherein the CCD scanner is a Time Delay Integration (TDI) type CCD scanner.

18. The method of claim 16 or 17, wherein forming the image of each sub-array includes overlapping at least one spot column of the sub-arrays using a plurality of images of adjacent sub-arrays.

19. The method of claim 17 or 18, further comprising determining a position of the main array by performing global alignment using a plurality of global alignment spot arrays separated from the plurality of sub-array alignment spot arrays to determine a position of the main array before repeatedly determining the position of each sub-array.

20. The method of any of claims 16 to 19, wherein repeatedly determining the position of each sub-array is based on spaces between each of the sub-arrays and wherein the CCD scanner is a step-and-repeat type CCD scanner.

21. The hybridization analysis method of claim 20, wherein forming the image of each of the sub-arrays includes overlapping at least one spot column of the sub-arrays and at least one spot row of the sub-arrays using a plurality of images of adjacent sub-arrays.

22. The hybridization analysis method of claim 20 or 21, further comprising determining a position of a main array of the oligomer probe array chip by performing global alignment using a plurality of global alignment spot arrays outside the main array before repeatedly determining the position of each of the sub-arrays.
